# EUROPÄISCHE PATENTSCHRIFT

(11) **EP 1 094 845 B1**
(45) Veröffentlichungstag und Bekanntmachung des Hinweises auf die Patenterteilung: **08.01.2003**
(21) Anmeldenummer: 99920739.2
(22) Anmeldetag: 22.04.1999
(51) Int. Cl.: A61L 2/06

(54) **VORRICHTUNG ZUR DAMPFSTERILISATION**
VAPOR STERILIZATION DEVICE
DISPOSITIF DE STERILISATION A LA VAPEUR

(30) Priorität: 09.07.1998 DE 19830706
(43) Veröffentlichungstag der Anmeldung: 02.05.2001
(73) Patentinhaber: KNF Neuberger GmbH, D-79112 Freiburg (DE)
(72) Erfinder: RIEDLINGER, Heinz, D-28211 Bremen (DE)
(74) Vertreter: Patent- und Rechtsanwaltssozietät, Maucher, Börjes & Kollegen
(86) Internationale Anmeldenummer: EP9902710
(87) Internationale Veröffentlichungsnummer: WO00002594

(56) Entgegenhaltungen:
- EP-A- 0 799 637
- WO-A-92/09310
- WO-A-98/28540
- DE-A- 2 540 407

## Beschreibung

Die Erfindung betrifft eine Vorrichtung zur Dampfsterilisation, die eine Sterilisationskammer sowie eine daran angeschlossene Vakuumpumpe aufweist, wobei der Saugeinlaß, der Verdichtungsraum sowie der Druckauslaß der Vakuumpumpe als Auslaßkanal für aus der Sterilisationskammer bei der Entspannung ausströmenden Dampf oder dergleichen Ausströmmedium dient.

Medizinische Instrumente und andere medizinische Gebrauchsgegenstände werden in Autoklaven der eingangs erwähnten Art sterilisiert, die eine luftdicht verschließbare Sterilisationskammer aufweisen. Dabei werden die in der Sterilisationskammer befindlichen Instrumente vor dem Sterilisationsvorgang zunächst einem sogenannten fraktionierten Vorvakuum ausgesetzt, bei dem durch wiederholtes Evakuieren der Luft im Wechsel mit dem periodischen Einströmen von Dampf eine besonders gute Luftentfernung auch aus englumigen Instrumenten erreicht wird. Während des Sterilisationsvorganges werden die Instrumente in der Sterilisationskammer unter Überdruck heißem Wasserdampf ausgesetzt. Um die Instrumente nach dem Sterilisieren rasch und rückstandslos zu trocknen, wird in der Sterilisationskammer anschließend mittels der Vakuumpumpe wiederum ein sogenanntes Nachvakuum erzeugt, welches die Trocknungszeit des Sterilisiergutes verkürzen und den Trocknungsvorgang optimieren soll.

Die jedem Sterilisationsvorgang nachfolgende Entspannung des in der Sterilisationskammer vorhandenen Dampfdruckes bis zum Atmosphärendruck erfolgt bei herkömmlichen Autoklaven parallel zur Vakuumpumpe durch einen separaten, mit der Atmosphäre verbundenen Auslaß, der über ein Elektromagnetventil gesteuert wird. Wenn der Atmosphärendruck erreicht ist, wird dieser Auslaß über das zugeordnete Elektromagnetventil geschlossen und die Sterilisationskammer zum nachfolgenden Evakuieren mit der Vakuumpumpe verbunden.

Der zum Entspannen des Dampfdruckes erforderliche zusätzliche Auslaß und das zugeordnete Elektromagnetventil erfordern jedoch einen nicht unwesentlichen konstruktiven Mehraufwand.

Aus der WO 92/09310 ist ein Verfahren zur Sterilisation hitzeempfindlicher Materialien bekannt, bei dem die in einem Autoklaven befindliche Luft in einem ersten Verfahrensschritt derart gegen überhitzten Wasserdampf ausgetauscht wird, daß der Autoklav zur Vermeidung einer Kondensatbildung vorgeheizt wird, daß das hitzeempfindliche Material in einem zweiten Verfahrensschritt unter Feuchtigkeitseinwirkung sterilisiert wird, und daß in einem dritten Verfahrensschritt der Dampf aus dem Autoklaven entfernt und das Material getrocknet wird.

Dabei wird in der WO 92/09310 auf Seite 9, letzter Absatz und Seite 10, erster Absatz, beiläufig beschrieben, daß der Dampf-Überdruck nach Beendigung der zweiten Verfahrensstufe durch öffnen eines Hahnes oder durch Einschalten einer Vakuumpumpe entfernt werden kann. In diesem Zusammenhang wird in der WO 92/09310 erwähnt, daß es zwar nicht zwingend erforderlich,jedoch möglich sei, den Dampf vor dem Einsaugen in die Vakuumpumpe zu kühlen.

Da die WO 92/09310 nur ein neues Sterilisationsverfahren für hitzeempfindliche Materialien, nicht aber einen weiterentwickelten und in seiner Leistung verbesserten Autoklaven beschreibt, sind der WO 92/09310 keine weiteren Ausführungen zum Autoklaven selbst und zu dessen Leistungsfähigkeit zu entnehmen.

Tatsächlich ist die Kühlung des Wasserdampfs nicht zwingend erforderlich, wenn statt dessen ein Aufheizen des Pumpenkopfes in Kauf genommen wird. Ein derartiges Aufheizen des Pumpenkopfes hat jedoch den Nachteil, daß die aus dem Autoklaven abgepumpte Feuchtigkeit verdampft, daß diese Verdampfung eine nicht unerhebliche Volumenvergrößerung des abzupumpenden Volumens nach sich zieht, und daß innerhalb der während der Taktzyklen vorgegebenen Zeitspanne dementsprechend nicht ein ausreichend großes Volumen abgepumpt werden kann, um das erwünscht hohe Vakuum zu erzeugen.

Es besteht daher die Aufgabe, eine Vorrichtung zur Dampfsterilisation der eingangs erwähnten Art zu schaffen, die mit einem vergleichsweise geringen Aufwand herstellbar ist und die sich dennoch durch eine hohe Leistungsfähigkeit insbesondere hinsichtlich des erreichbaren Vakuums auszeichnet.

Die erfindungsgemäße Lösung dieser Aufgabe besteht bei der Vorrichtung der eingangs erwähnten Art insbesondere darin, daß zumindest ein Teil der mit dem Ausströmmedium dabei in Kontakt kommenden Teile der Vakuumpumpe aus schlecht wärmeleitendem Werkstoff bestehen.

Bei der erfindungsgemäßen Vorrichtung zur Dampfsterilisation erfolgt die Entspannung des in der Sterilisationskammer vorhandenen Dampfdruckes durch die Vakuumpumpe. Dadurch kann auf einen zusätzlichen Auslaß und das erforderliche Elektromagnetventil verzichtet werden. Beim Entspannen des in der Sterilisationskammer vorhandenen Dampfdruckes strömt zwar der heiße Wasserdampf durch den Pumpenkopf, - da jedoch bei der erfindungsgemäßen Pumpe zumindest ein Teil der mit dem Ausströmmedium dabei in Kontakt kommenden Teile der Vakuumpumpe aus schlecht wärmeleitendem Werkstoff bestehen, können die zur Erzielung des notwendigen Vakuums erforderlichen Pumpenkopf-Temperaturen niedrig gehalten werden, ohne daß eine besonders aufwendige Luft- oder Wasserkühlung der Pumpe erforderlich wäre.

Mit Hilfe der aus einem schlecht wärmeleitendem Werkstoff bestehenden Pumpenteile wird verhindert, daß sich die den Verdichtungsraum begrenzenden Pumpenteile durch den Dampfstoß wesentlich aufheizen können.

Die erfindungsgemäße Lösung eignet sich vor allem für zyklische Dampfsterilisationsverfahren, da bei der relativ kurzen Aufheizzeit die Temperatur der Kunststoffteile nur geringfügig zunimmt, so daß die Kunststoffteile beim nachfolgenden Durchströmen des Kondensats durch den Pumpenkopf wieder abgekühlt werden.

Damit die Vakuumpumpe der erfindungsgemäßen Vorrichtung während des Entspannungsvorganges praktisch leer mitlaufen kann, um anschließend, nach Erreichen des atmosphärischen Druckes, automatisch mit dem Evakuieren zu beginnen, ist es vorteilhaft, wenn die in Saugbetrieb befindliche Vakuumpumpe über ihren Saugeinlaß, den Verdichtungsraum sowie den Druckauslaß als Auslaßkanal für aus der Sterilisationskammer bei der Entspannung ausströmenden Dampf dient.

Um die den Verdichtungsraum begrenzenden Pumpenteile aus einem schlecht wärmeleitenden Werkstoff herstellen zu können, sieht eine bevorzugte Ausführungsform gemäß der Erfindung vor, daß die mit dem Ausströmmedium in Kontakt kommenden Teile der Vakuumpumpe aus Kunststoff bestehen. Mittels dieser aus Kunststoff hergestellten Pumpenteile wird verhindert, daß sich die den Verdichtungsraum begrenzenden Teile durch den beim Entspannen des Dampfdruckes durchströmenden Dampfstoß wesentlich aufheizen können.

Eine bevorzugte Ausführungsform gemäß der Erfindung sieht vor, daß die Vakuumpumpe als Membranpumpe ausgebildet ist. Um auf einen zusätzlichen Auslaß und das dazugehörige Elektromagnetventil verzichten zu können, ist der Pumpenkopf dieser Membranpumpe vorzugsweise aus einem schlecht wärmeleitenden Material, insbesondere aus Kunststoff, hergestellt.

## Patentansprüche

1. Vorrichtung zur Dampfsterilisation, die eine Sterilisationskammer sowie eine daran angeschlossene Vakuumpumpe aufweist, wobei der Saugeinlaß, der Verdichtungsraum sowie der Druckauslaß der Vakuumpumpe als Auslaßkanal für aus der Sterilisationskammer bei der Entspannung ausströmenden Dampf oder dergleichen Ausströmmedium dient, **dadurch gekennzeichnet, daß** zumindest ein Teil der mit dem Ausströmmedium dabei in Kontakt kommenden Teile der Vakuumpumpe aus schlecht wärmeleitendem Werkstoff bestehen.

2. Vorrichtung nach Anspruch 1, **dadurch gekennzeichnet, daß** die in Saugbetrieb befindliche Vakuumpumpe über ihren Saugeinlaß, den Verdichtungsraum sowie den Druckauslaß als Auslaßkanal für aus der Sterilisationskammer bei der Entspannung ausströmenden Dampf oder dergleichen Ausströmmedium dient.

3. Vorrichtung nach Anspruch 1 oder 2, **dadurch gekennzeichnet, daß** mit dem Ausströmmedium in Kontakt kommende Teile der Vakuumpumpe aus Kunststoff bestehen.

4. Vorrichtung nach einem der Ansprüche 1 bis 3, **dadurch gekennzeichnet, daß** die Vakuumpumpe als Membranpumpe ausgebildet ist.

5. Vorrichtung nach Anspruch 4, **dadurch gekennzeichnet, daß** der Pumpenkopf der Membranpumpe aus einem schlecht wärmeleitenden Werkstoff, insbesondere aus Kunststoff, besteht.

## Claims

1. Apparatus for steam sterilization which comprises a sterilization chamber and a vacuum pump attached thereto, wherein the suction inlet, the compression chamber and the pressure outlet of the vacuum pump serve as an outlet channel for steam or a similar outflow medium flowing out of the sterilization chamber when the pressure is relieved, **characterised in that** at least some of the parts of the vacuum pump coming into contact with the outflow medium consist of a material which is a poor conductor of heat.

2. Apparatus according to claim 1, **characterised in that** the vacuum pump operation on suction acts as an outlet channel for steam or a similar outflow medium flowing out of the sterilization chamber during pressure relief, by means of its suction inlet, the compression chamber and the pressure outlet.

3. Apparatus according to claim 1 or 2, **characterised in that** parts of the vacuum pump coming into contact with the outflow medium consists of plastics.

4. Apparatus according to one of claims 1 to 3, **characterised in that** the vacuum pump is constructed as a diaphragm pump.

5. Apparatus according to claim 4, **characterised in that** the pump head of the diaphragm pump consists of a material which is a poor conductor of heat, particularly plastic.

## Revendications

1. Dispositif de stérilisation à la vapeur, qui présente une chambre de stérilisation et une pompe à vide raccordée à cette chambre, l'entrée d'aspiration, la chambre de compression et la sortie de refoulement de la pompe à vide servant de canal d'évacuation pour la vapeur ou fluide analogue évacué de la chambre de stérilisation lors de la détente, **caractérisé en ce qu'**au moins une partie des éléments de la pompe à vide qui entrent alors en contact avec le fluide évacué sont réalisés en matériau mauvais conducteur thermique.

2. Dispositif selon la revendication **1, caractérisé en ce que** la pompe à vide se trouvant en fonctionnement d'aspiration sert, par l'intermédiaire de son entrée d'aspiration, de la chambre de compression et de la sortie de refoulement, de canal d'évacuation pour la vapeur ou fluide analogue évacué de la chambre de stérilisation lors de la détente.

3. Dispositif selon la revendication 1 ou 2, **caractérisé en ce que** les éléments de la pompe à vide qui entrent en contact avec le fluide évacué sont réalisés en matière plastique.

4. Dispositif selon l'une quelconque des revendications 1 à 3, **caractérisé en ce que** la pompe à vide est réalisée sous forme de pompe à membrane.

5. Dispositif selon la revendication 4, **caractérisé en ce que** la tête de pompe de la pompe à membrane est réalisée en un matériau mauvais conducteur thermique, notamment en matière plastique.
